**Europäisches Patentamt**

**European Patent Office**

**Office européen des brevets**

(19)

(11) Veröffentlichungsnummer: **0 083 708**
**B1**

# (12) EUROPÄISCHE PATENTSCHRIFT

(45) Veröffentlichungstag der Patentschrift:
**19.06.85**

(51) Int. Cl.⁴: **A 61 F 2/30**

(21) Anmeldenummer: **82110498.1**

(22) Anmeldetag: **13.11.82**

(54) **Zementfrei im Becken verankerbare Gelenkpfanne.**

(30) Priorität: **08.01.82 CH 91/82**

(43) Veröffentlichungstag der Anmeldung:
**20.07.83 Patentblatt 83/29**

(45) Bekanntmachung des Hinweises auf die Patenterteilung:
**19.06.85 Patentblatt 85/25**

(84) Benannte Vertragsstaaten:
**AT BE DE FR GB IT NL**

(56) Entgegenhaltungen:
**FR - A - 2 301 217**
**FR - A - 2 416 004**
**FR - A - 2 429 009**

(73) Patentinhaber: **GEBRÜDER SULZER
AKTIENGESELLSCHAFT, Zürcherstrasse 9,
CH-8401 Winterthur (CH)**

(72) Erfinder: **Schreiber, Adam, Prof. Dr.-med.,
Glärnischstrasse 10, CH-8700 Küsnacht (CH)**
Erfinder: **Hilaire, Jacob, Stadlerstrasse 168,
CH-8404 Winterthur (CH)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

## Beschreibung

Die Erfindung betrifft eine zementfrei im Bekken verankerbare Gelenkpfanne zur Aufnahme des Gelenkkopfes einer Hüftgelenkprothese, wobei die Außenform der Pfanne einen rotationssymmetrischen Kegelstumpf bildet.

Für eine zementfreie Verankerung sind künstliche Gelenkpfannen der vorstehend genannten Art für Totalprothesen des Hüftgelenks bekannt (CH-A-568 753); die Form des Kegelstumpfes ist dabei gewählt worden, weil sich gezeigt hat, daß die ebenfalls bekannten halbkugelschalenförmigen Pfannen — selbst wenn ihre äußere Halbkugelfläche mit zirkularen, gegebenenfalls durch längs einer Mantellinie von Rillen unterbrochenen Rippen und Vertiefungen versehen ist (FR-A-2 301 217) —, besonders bei der angestrebten zementfreien Verankerung, durch elstische Verformungen infolge der wechselnden Be- und Entlastungen des Beckenknochens relativ leicht aus ihrer Verankerung herausgedrückt werden.

Zur Fixierung im Beckenknochen ist in den Mantel des Kegelstumpfes bei den bekannten kegelstumpfförmigen Pfannen ein Gewinde eingeschnitten, mit dem die Pfanne in ein entsprechendes Gewinde in das künstlich geschaffene Pfannenlager des Beckenknochens eingeschraubt wird.

Die Fixierung dieser bekannten Pfanne mit Hilfe eines Gewindes hat sich in der Praxis als nachteilig erwiesen, da das Schneiden eines Gewindes mit der für eine zementfreie Fixierung erforderlichen Genauigkeit in das operativ geschaffene kegelstumpfförmige Pfannenlager im Beckenknocken erhebliche Schwierigkeiten bereitet. Weiterhin besteht die Gefahr, daß das in den relativ weichen Knochen eingeschnittene Gewinde, beispielsweise durch »Überdrehen« des Gewindeschneiders, beschädigt oder wieder zerstört wird.

Aus der FR-A-2 416 004 ist eine zweiteilige Hüftgelenkspfanne bekannt, die ohne Schraubbewegung in den Knochen eingetrieben wird; ihr äußerer aufzuweitender Teil ist eine im Polbereich geschlossene Kappe.

Beim Einschlagen des Einsatzes erfährt dieser eine Aufweitung, die nach allen Seiten gleichmäßig um die Rotationssymmetrie-Achse der Kappe herum erfolgt. Die Oberfläche und damit der äußere Rand der Kappe bewegen sich dabei sowohl in radialer als auch in axialer Richtung. Eine fixierende Klemmwirkung wird dadurch nur längs des Randes erreicht. Weiterhin sind die »Eindringtiefe« des Einsatzes und damit die Aufweitmöglichkeit infolge des geschlossenen Polbereichs begrenzt.

Aufgabe der Erfindung ist es, eine Gelenkpfanne für eine zementfreie Verankerung zu schaffen, bei deren Implantation auf der einen Seite das Schneiden eines Gewindes in den Beckenknochen entfällt, und auf der anderen Seite durch eine radiale Aufweitung die Fixierung einer ohne Drehung eingetriebenen Pfannenprothese verbessert ist.

Erfindungsgemäß wird diese Aufgabe gelöst durch eine einseitig geschlitzte Hülse, die längs einer Mantellinie verlaufende Rillen und zirkulare Vertiefungen trägt, zwischen denen in Umfangsrichtung verlaufende Querrippen verbleiben, und deren Innenhohlraum ein sich in der gleichen Richtung wie der äußere Kegelstumpf verjüngender Konus ist, und ferner durch einen, die eigentliche Pfannenschale enthaltenden konischen Einsatz, der in den Innenhohlraum der Hülse einpreßbar ist.

Für die Fixierung dieser neuen Pfanne muß im Knochen ein Pfannenlager ohne Gewinde, beispielsweise durch Fräsen, hergestellt werden. In dieses wird dann die Hülse, deren Durchmesser infolge des Schlitzes durch Zusammenpressen in Umfangsrichtung etwas — beispielsweise etwa 2 mm — verkleinert werden kann, in Richtung ihrer Achse einfach eingesetzt. Die Hülse ist dabei mit Vorteil in Umfangsrichtung elastisch ausgebildet. Anschließend steckt man den Einsatz in den Innenhohlraum der Hülse und preßt oder schlägt ihn leicht ein. Hierdurch erfolgt ein Aufspreizen der geschlitzten Hülse, wobei die Querrippen der Hülse in die Wand des Pfannenlagers »eindringen« können.

Durch die radiale Aufweitung entsteht dabei eine weitgehend auf der Mantelfläche — und nicht nur längs einer Linie wirkende — radiale Spannung.

Auf diese Weise wird die sogenannte primäre Fixierung gewährleistet, durch die die Pfanne gehalten werden muß, bis an sie Gewebe an- bzw. in ihre Vertiefungen eingewachsen ist; die Rillen, die in Umfangsrichtung zwischen den einzelnen Vertiefungen vorhanden sind, übernehmen dabei die Aufgabe einer Verdrehsicherung. Vorteilhafterweise ragen sie daher tiefer in den Hülsenkörper hinein als die Vertiefungen. Weiterhin kann die Haftung des Einsatzes im Innenhohlraum der Hülse verbessert werden, wenn beide Konen selbsthemmend sind.

Um ein zu tiefes Einpressen bzw. Eindringen der Pfanne in den Knochen zu verhindern, kann es zweckmäßig sein, wenn das weite Ende der Hülse durch einen als Anschlag wirkenden Rand abgeschlossen ist.

Um ein Eindringen der Querrippen in das Knochengewebe bei einer bestimmten, durch den eingetriebenen Einsatz gegebenen »Sprengkraft« zu gewährleisten, sind bekanntlich unter Umständen für unterschiedliche Materialien unterschiedliche Werte für das Verhältnis Rippenhöhe/Rippenbreite bzw. Tiefe/gegenseitiger Abstand der Vertiefungen erforderlich; daher ist es zweckmäßig, wenn die Tiefe der Vertiefungen und der Abstand zweier benachbarter Vertiefungen in Abhängigkeit vom Material der Hülse so gewählt sind, daß die Querrippen in den Knochen eindringen.

Bei der Dimensionierung einer Hülse aus Polyäthylen hat es sich beispielsweise als vorteilhaft erwiesen, wenn die Tiefe der Vertiefungen 1 bis 4 mm und ihr Abstand von Sohle zu Sohle 2 bis 5 mm betragen; obwohl körperverträgliche und -beständige Kunststoffe die bevorzugten Werkstoffe für die neue Pfanne, insbesondere für ihre Hülse sind, kann diese Hülse auch aus einem in der Implantattechnik üblichen Metall oder einer Metall-Legierung gefertigt werden. Für den Einsatz eignen sich alle in der Implantattechnik üblichen Werkstoffe, also Kunststoffe, Metalle, Metallegierungen und biokeramische Materialien.

Im folgenden wird die Erfindung anhand eines Ausführungsbeispiels im Zusamenhang mit der Zeichnung näher erläutert.

Fig. 1 ist eine Seitenansicht einer Ausführungsform der neuen Gelenkpfanne in auseinandergezogener Darstellung;

Fig. 2 ist der Schnitt II-II durch die Hülse von Fig. 1.

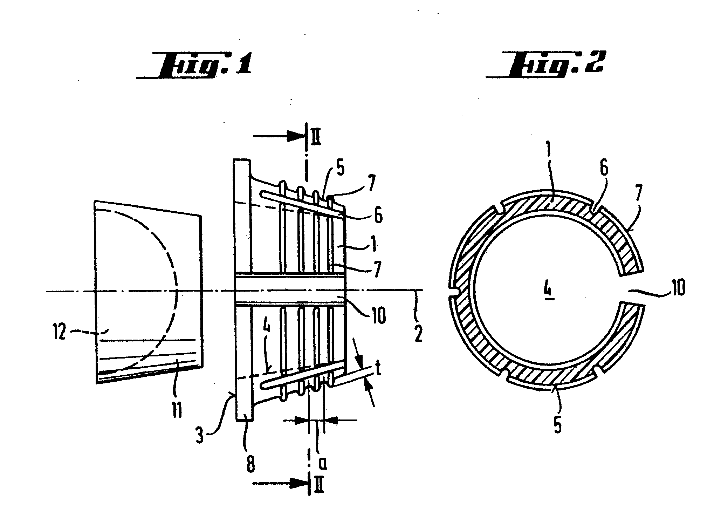

Die Gelenkpfanne besteht aus einer Hülse 1 (Fig. 1) und einem konischen Einsatz 11; die Hülse 1 ist ein rotationssymmetrischer Kegelstumpf, dessen Symmetrieachse mit 2 bezeichnet ist. Sie hat in ihrem Umfang einen Schlitz 10, dessen Mindestbreite so groß ist, daß der Hülsendurchmesser durch Zusammendrücken in Umfangsrichtung elastisch um einige Millimeter verringert werden kann.

In die Mantelfläche der Hülse 1 sind Vertiefungen 5 eingearbeitet, zwischen denen entlang einer Mantellinie verlaufende Rillen 6 vorhanden sind; diese dienen, wie erwähnt, als Verdrehsicherung.

Zwischen den parallel zueinander in Umfangsrichtung verlaufenden Vertiefungen 6 verbleiben Querrippen 7; deren Höhe und Abstand sind materialabhängig so gewählt, daß sich mindestens ein Teil von ihnen beim Einpressen der Pfanne bzw. Hülse in den vorbereiteten Beckenknochen elastisch verformt und somit die primäre Fixierung fördernde, widerhakenartige Verankerungen an der Knochenwand bilden.

Am weiteren Ende 3 hat die Hülse 1 einen über ihre Mantelfläche vorstehenden Rand 8, der als Anschlag beim Einsetzen in den Knochen dient.

Koaxial zum Mantel durchsetzt ein konischer Innenhohlraum 4 die Hülse 1; dieser dient zur paßgenauen Aufnahme des Einsatzes 11, aus dem ebenfalls vom weiten Ende her und wiederum koaxial, eine halbkugelförmige Pfannenschale 12 für die Aufnahme des Gelenkkopfes einer nicht dargestellten Femurprothese herausgearbeitet ist.

Wie erwähnt, besteht die neue zweiteilige Gelenkpfanne vorzugsweise aus Polyäthylen; für dieses Material beträgt die Tiefe t der Vertiefungen 5 beispielsweise 2 mm, während ihr Abstand a, gemessen von Sohle zu Sohle, 3 mm ist. Der halbe Konuswinkel des Einsatzes 11 bzw. des Innenhohlraumes 4 — gemessen gegen die Rotationsachse 2 — ist vorzugsweise selbsthemmend und hat einen Wert von 5°.

Für die Implantation der neuen Pfanne hat sich beispielsweise als zweckmäßig erwiesen, im Knochen eine in Form und Abmessungen an die Hülse 1 paßgenau angepaßte Ausnehmung zu schaffen, die gegebenenfalls sogar ein wenig enger als die Außenform der »entspannten«, d. h. nicht zusammengepreßten Hülse 1 sein kann; anschließend wird die Hülse 1 zusammengedrückt und in diese Ausnehmung eingesetzt. Nunmehr wird der Einsatz 11 in den Innenhohlraum 4 eingepreßt und leicht eingeschlagen; durch ihn wird die Hülse 1 aufgeweitet und mit ihren Rippen 7 in die Ausnehmung gepreßt.

Da die Form der ganzen Gelenkpfanne, einschließlich derjenigen der eigentlichen Pfannenschale 12, vollkommen rotationssymmetrisch ist, kann die Pfanne bei der Implantation um ihre Achse beliebig gedreht und ohne Beachtung einer bestimmten Lage — beispielsweise einer markierten Stelle des Randes der Hülse 1 relativ zu einem Bezugspunkt am Beckenknochen — eingesetzt werden, was ein weiterer Vorteil der neuen Konstruktion ist.

Die erfindungsgemäße Aufweitung in Verbindung mit der Ausbildung der Mantelfläche der Hülse 1 gewährleistet eine sichere Primär-Fixierung der Pfanne im Knochen, während ihre Kegelstumpfform gleichzeitig eine großflächige Abstützung im Knochen ermöglicht, die den elastischen Verformungen des Beckens bei Be- und Entlastungen in weitem Umfang nachkommen kann.

**Patentansprüche**

1. Zementfrei im Becken verankerbare Gelenkpfanne zur Aufnahme des Gelenkkopfes einer Hüftgelenkprothese, wobei die Außenform der Pfanne einen rotationssymmetrischen Kegelstumpf bildet, gekennzeichnet durch eine einseitig geschlitzte Hülse (1), die längs einer Mantellinie verlaufende Rillen (6) und zirkulare Vertiefungen (5) trägt, zwischen denen in Umfangsrichtung verlaufende Querrippen (7) verbleiben, und deren Innenhohlraum (4) ein sich in der gleichen Richtung wie der äußere Kegelstumpf verjüngender Konus ist, und ferner durch einen, die eigentliche Pfannenschale (12) enthaltenden konischen Einsatz (11), der in den Innenhohlraum (4) der Hülse (1) einpreßbar ist.

2. Gelenkpfanne nach Anspruch 1, dadurch gekennzeichnet, daß der innere Konus der Hülse (1) und der Konus des Einsatzes (11) selbsthemmend ausgebildet sind.

3. Gelenkpfanne nach Anspruch 1, dadurch gekennzeichnet, daß die Tiefe (t) der Vertiefungen (5) und der Abstand (a) zweier benachbarter Vertiefungen (5) in Abhängigkeit vom Material der Hülse (1) so gewählt sind, daß die Querrippen (7) in den Knochen eindringen.

4. Gelenkpfanne nach Anspruch 1, dadurch gekennzeichnet, daß das weite Ende (3) der Hülse (1) durch einen als Anschlag wirkenden Rand (8) abgeschlossen ist.

5. Gelenkpfanne nach Anspruch 1, dadurch gekennzeichnet, daß die Hülse (1) in Umfangsrichtung elastisch verformbar ist.

6. Gelenkpfanne nach Anspruch 3, deren Hülse aus Polyäthylen der Klassifikationen HDPE oder UHMW besteht, dadurch gekennzeichnet, daß die Tiefe (t) der Vertiefungen (5) 1—4 mm und ihr Abstand (a) von Sohle zu Sohle 2—5 mm betragen.

7. Gelenkpfanne nach Anspruch 1, dadurch gekennzeichnet, daß die Rillen (6) tiefer in den Hülsenkörper hineinreichen als die Vertiefungen (5).

**Claims**

1. An acetabulum adapted to receive the fermoral head of a prosthetic hip joint, the acetabulum being anchorable in the basin without cement, the outside shape of the acetabulum being a frustum which is symmetrical in rotation, characterised by: a sleeve (1) which is slotted on one side and which is formed with grooves (6) extending along a generatrix and which has circular recessed parts (5), transverse ribs (7) which around the periphery remaining between the recessed parts (5), the hollow interior (4) of the sleeve (5) being a cone which narrows in the same direction as the outer frustum; and a conical insert (11) which contains the acetabulum socket (12) and which is adapted to be pressed into the sleeve interior (4).

2. An acetabulum according to claim 1, characterised in that the inner cone of the sleeve (1) and the cone of the insert (11) are self-locking.

3. An acetabulum according to claim 1, characterised in that the depth (t) of the recessed parts (5) and the distance (a) between two adjacent recessed parts (5) are so chosen in dependence upon the sleeve material that the transverse ribs (7) penetrate into the bone.

4. An acetabulum according to claim 1, characterised in that the wide end (3) of the sleeve (1) is closed by an edge (8) acting as abutment.

5. An acetabulum according to claim 1, characterised in that the sleeve (1) is resiliently deformable in the peripheral direction.

6. An acetabulum according to claim 3 whose sleeve is made of class HDPE or UHMW polythene, characterised in that the depth (t) of the recessed parts (5) is from 1 to 4 mm and their between-centres spacing (a) is from 2 to 5 mm.

7. An acetabulum according to claim 1, characterised in that the grooves (6) extend deeper into the sleeve body the recessed parts (5).

**Revendications**

1. Cuvette d'articulation pouvant être ancrée sans ciment dans le bassin, pour recevoir la tête d'articulation d'une prothèse coxo-fémorale, la configuration externe de la cuvette formant un tronc de cône à symétrie de révolution, caractérisé par une douille (1) fendue d'un côté, comportant des gorges (6) et des renfoncements circulaires (5) s'étendant le long d'une génératrice, entre lesquels subsistent des nervures transversales (7) s'étendant dans le sens circonférentiel, et dont la cavité interne (4) est un cône décroissant de section dans la même direction que le tronc de cône externe; ainsi que par une pièce insérée tronconique (11) qui renferme la calotte (12) de la cuvette proprement dite et peut être introduite par pression dans la cavité interne (4) de la douille (1).

2. Cuvette d'articulation selon la revendication 1, caractérisé par le fait que le cône interne de la douille (1) et le cône de la pièce insérée (11) sont réalisés avec effet d'auto-blocage.

3. Cuvette d'articulation selon la revendication 1, caractérisé par le fait que la profondeur (t) des renfoncements (5) et l'espacement (a) entre deux renfoncements voisins (5) sont choisis, en fonction du matériau constituant la douille (1), de telle sorte que les nervures transversales (7) pénètrent dans l'os.

4. Cuvette d'articulation selon la revendication 1, caractérisé par le fait que l'extrémité large (3) de la douille (1) s'achève par un rebord (8) faisant office de butée.

5. Cuvette d'articulation selon la revendication 1, caractérisé par le fait que la douille (1) est déformable élastiquement dans le sens périphérique.

6. Cuvette d'articulation selon la revendication 3, caractérisé par le dont la douille consiste en du polyéthylène des classifications HDPE ou UHMW, caractérisé par le fait que la profondeur (t) des renfoncements (5) est de 1—4 mm, et leur espacement (a) de creux à creux est de 2—5 mm.

7. Cuvette d'articulation selon la revendication 1, caractérisé par le fait que les gorges (6) pénètrent plus profondément que les renfoncements (5) dans le corps de la douille.

# Fig. 1

# Fig. 2